# EUROPEAN PATENT SPECIFICATION

(11) **EP 1 606 628 B1**
(45) Date of publication and mention of the grant of the patent: **25.10.2006**
(21) Application number: 04722562.8
(22) Date of filing: 23.03.2004
(51) Int. Cl.: G01N 33/68, C07K 14/47

(54) **PH-DEPENDENT POLYPEPTIDE AGGREGATION AND ITS USE**
PH-ABHÄNGIGE POLYPEPTIDAGGREGATION UND VERWENDUNG DAVON
AGREGATION DE POLYPEPTIDES DEPENDANTE DU PH ET SON UTILISATION

(30) Priority: 25.03.2003 US 397059
(43) Date of publication of application: 21.12.2005
(73) Proprietor: Eidgenoessische Technische Hochschule Zuerich, 8092 Zürich (CH)
(72) Inventor: ZAHN, Ralph, CH-8049 Zürich (CH)
(74) Representative: Kasche, André
(86) International application number: PCT/EP2004/003060
(87) International publication number: WO 2004/085464

(56) References cited:
- WO-A-01/35104
- WO-A-01/77687
- US-A- 5 773 572
- CALLAHAM M A ET AL: "REVERSIBILITY OF SCRAPIE-ASSOCIATED PRION PROTEIN AGGREGATION" JOURNAL OF BIOLOGICAL CHEMISTRY, AMERICAN SOCIETY OF BIOLOGICAL CHEMISTS, BALTIMORE, MD, US, vol. 276, no. 30, 27 July 2001 (2001-07-27), pages 28022-28028, XP001094078 ISSN: 0021-9258
- BURNS COLIN S ET AL: "Molecular features of the copper binding sites in the octarepeat domain of the prion protein" BIOCHEMISTRY, vol. 41, no. 12, 26 March 2002 (2002-03-26), pages 3991-4001, XP002291260 ISSN: 0006-2960 cited in the application
- WARNER R G ET AL: "Identification of the heparan sulfate binding sites in the cellular prion protein" JOURNAL OF BIOLOGICAL CHEMISTRY, AMERICAN SOCIETY OF BIOLOGICAL CHEMISTS, BALTIMORE, MD, US, vol. 277, no. 21, 24 May 2002 (2002-05-24), pages 18421-18430, XP002258986 ISSN: 0021-9258
- SABORIO GABRIELA P ET AL: "Sensitive detection of pathological prion protein by cyclic amplification of protein misfolding" NATURE, MACMILLAN JOURNALS LTD. LONDON, GB, vol. 411, no. 6839, 2001, pages 810-813, XP002201227 ISSN: 0028-0836 cited in the application
- ZOUEVA OLGA P ET AL: "Aggregation of influenza virus ribonucleocapsids at low pH" VIRUS RESEARCH, vol. 85, no. 2, 10 May 2002 (2002-05-10), pages 141-149, XP002291311 ISSN: 0168-1702
- ZAHN R: "The Octapeptide Repeats in Mammalian Prion Protein Constitute a pH-dependent Folding and Aggregation Site" JOURNAL OF MOLECULAR BIOLOGY, LONDON, GB, vol. 334, no. 3, 28 November 2003 (2003-11-28), pages 477-488, XP004472516 ISSN: 0022-2836

## Description

### BACKGROUND OF THE INVENTION

The prion protein (PrP) was detected in attempts to identify the infective agent of transmissible spongiform encephalopathies (TSE), and consequently we know a lot about the pathological activity of the scrapie form, PrP^{Sc}, whereas the physiological function of the cellular form, PrP^{C}, remains an enigma (Prusiner, S.B. (1998) Prions. *Proc Natl Acad Sci U S A* 95, 13363-13383). PrP^{C} is a synaptic glycoprotein with a heterogeneous distribution in healthy adult brain that is attached to the cell surface *via* a glycosyl phosphatidylinositol (GPI) anchor and partitions to membrane domains that have been termed lipid rafts. The localization of PrP^{C} on the cell surface suggests that it may function in cell adhesion, ligand uptake or transmembrane signaling.

Based on biochemical analyses of chicken PrP^{C}, it was hypothesized that PrP^{C} might be involved in regulating the expression of cholinergic receptors at synaptic endings. Indeed immunohistochemistry of PrP^{C}-overexpressing transgenic mice revealed a synaptic expression pattern with PrP^{C} being predominantly located in the synaptic plasma membrane and, to a lesser extent, in synaptic vesicles. Electron microscopy showed that the protein is present both pre- and postsynaptically. PrP^{C} has also been localized along elongating axons, and there is increasing evidence that PrP^{C} may play a role in the growth of axons perhaps as an adhesion protein.

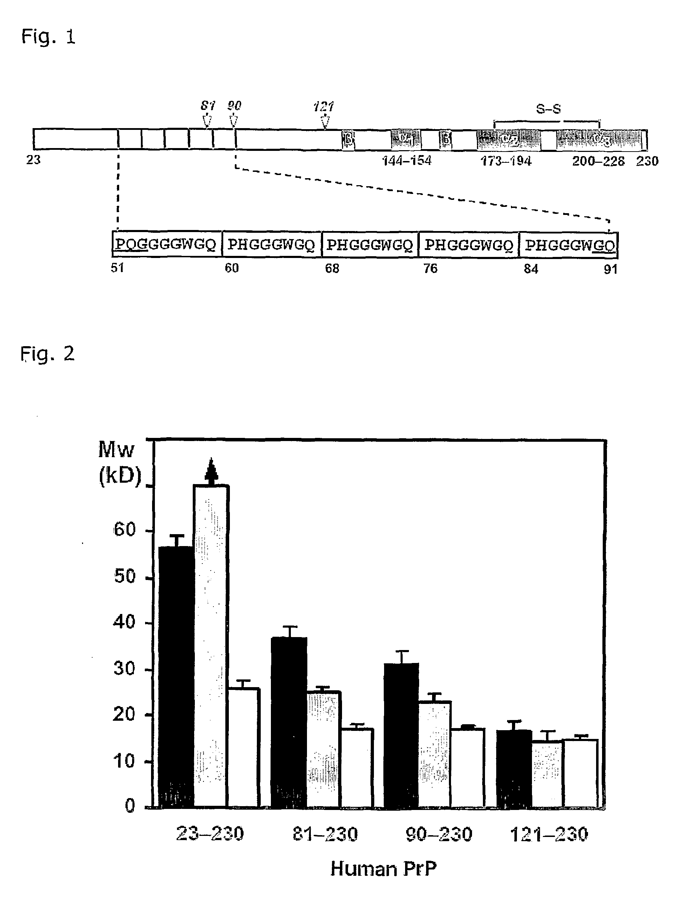

The octapeptide repeat region, comprised of repeats of the sequence PHGGGWGQ, is among the most conserved segments of PrP in mammals (Schätzl, H.M., Da Costa, M., Taylor, L., Cohen, F.E. and Prusiner, S.B. (1995) Prion protein gene variation among primates. *J Mol Biol* 245, 362-374; Wopfner, F., Weidenhofer, G., Schneider, R., von Brunn, A., Gilch, S., Schwarz, T.F., Werner, T. and Schätzl, H.M. (1999) Analysis of 27 mammalian and 9 avian PrPs reveals high conservation of flexible regions of the prion protein. *J Mol Biol* 289, 1163-1178). Residues 60 to 91 in human PrP consist of four His containing octapeptide repeats (OPR), and residues 51 to 59 consist of the homologous sequence PQGGGGWGQ (Figure 1).

Figure 1 shows the primary structure of the human prion protein (hPrP). The mature human prion protein consists of residues 23 to 230. The detailed amino acid sequence of the OPR region of residues 51 to 91 (gray boxes) is shown at the bottom, with residues unambiguously assigned in the nuclear magnetic resonance (NMR) spectra being underlined. For the segment 54-89 only a single set of resonance signals was detected for each repeated amino acid. Regular secondary structure elements are represented in black. The disulfide bond (S-S) between Cys179 and Cys214 is drawn as a gray line. Arrows at the top indicate N-terminal truncations sites of the hPrP constructs used in this study.

The binding of copper to the OPR of mammalian and avian prion proteins was first demonstrated by Hornshaw and co-workers (Hornshaw, M.P., McDermott, J.R., Candy, J.M. and Lakey, J.H. (1995) Copper binding to the N-terminal tandem repeat region of mammalian and avian prion protein: structural studies using synthetic peptides. *Biochem Biophys Res Commun* 214, 993-999; Hornshaw, M.P., McDermott, J.R., Candy, J.M. and Lakey, J.H. (1995) Copper-Binding to the N-Terminal Tandem Repeat Region of Mammalian and Avian Prion Protein - Structural Studies Using Synthetic Peptides. *Biochemical and Biophysical Research Communications* 214, 993-999), and it has been suggested that copper-binding is involved in the physiological function of PrP^{C} (Brown, D.R. et al. (1997) The cellular prion protein binds copper *in vivo. Nature* 390, 684-687). Recently, a heparin binding site has been identified within the OPR of PrP^{c}, where binding is enhanced in the presence of Cu²⁺. The finding that the laminin receptor protein acts as a receptor for PrP^{c} in the presence of heparan sulfate suggests a complex interaction between prion protein, copper, heparin/heparan sulfate, and receptor proteins with implications for the cellular function of prion proteins. It has also been suggested that PrP^{c} is released from synaptic vesicles to prevent unspecific copper binding of proteins in the synaptic cleft and that it supports the re-uptake of copper into the presynapse through endocytosis.

### PROBLEMS OBSERVED IN PRIOR ART

Transmissible spongiform encephalopathies (TSE) or prion diseases are fatal disorders of the central nervous system caused by unconventional infectious agents (prions) that are composed of a prion protein (PrP^{Sc}) (Prusiner, S.B. (1998) Prions. *Proc Natl Acad Sci U S A* 95, 13363-13383). The key event in TSE is the conformational change of a host protein, cellular prion protein (PrP^{C}), encoded by the prion gene PRNP, into the neuropathological isoform PrP^{Sc} that aggregates into amyloid fibrils and accumulates into neural and lymphoreticular cells (Doi, S., Ito, M., Shinagawa, M., Sato, G., Isomura, H. and Goto, H. (1988) Western blot detection of scrapie-associated fibril protein in tissues outside the central nervous system from preclinical scrapie-infected mice. *J Gen Virol* 69 (Pt 4), 955-960; Wadsworth, J.D.F., Joiner, S., Hill, A.F., Campbell, T.A., Desbruslais, M., Luthert, P.J. and Collinge, J. (2001) Tissue distribution of protease resistant prion protein in variant Creutzfeldt-Jakob disease using a highly sensitive immunoblotting assay. *Lancet* 358, 171-180). Diagnostic strategies used for the detection of other infectious agents, such as PCR, are therefore useless. However, accurate diagnostic methods at early stages of clinical signs or during the pre-clinical phase of the disease are needed as *in vivo* screening tests or the identification of infected individuals. These tests are not yet available, although enormous efforts have been made in the past years.

The formation of PrP^{Sc} occurs only in TSE and therefore is a specific marker for these disorders. Despite the wide distribution of PrP^{Sc} and infectivity in the body of TSE-affected hosts, the histological and biochemical lesions are restricted only to the central nervous system (CNS), termed spongiform encephalopathy. In sporadic and genetic TSE, the tissue where PrP^{Sc} originates is unknown, but it is likely that PrP^{Sc} starts in the CNS, thus making the development of early diagnostic methods based upon the detection of PrP^{Sc} in easily accessible tissues or body fluids useless. During infection with prions PrP^{Sc} is readily detectable in lymphoreticular tissues (Doi, S. et al. (1988) Western blot detection of scrapie-associated fibril protein in tissues outside the central nervous system from preclinical scrapie-infected mice. *J Gen Viral* 69 ( Pt 4), 955-960) leading to the suggestion of measuring PrP^{Sc} in tonsil tissue taken at biopsy for the diagnosis of scrapie in sheep and vCJD (Hill, A.F., Zeidler, M., Ironside, J. and Collinge, J. (1997) Diagnosis of new variant Creutzfeldt-Jakob disease by tonsil biopsy. *Lancet* 349, 99-100), a new variant of Creutzfeldt-Jakob disease that has been transmitted from BSE-infected cattle to human.

In recent years, great attention has been paid to the possible use of PrP^{Sc} detection in peripheral and accessible tissues (such as tonsil) or body fluids (such as the cerebrospinal fluid (CSF) or blood) for preclinical *in vivo* diagnostic of TSE. Experimental data failed to detect infectivity in blood of patients affected with any form of human TSE, but an exception could be vCJD patients where concern about the infectivity in blood has been raised, mostly because of the high level of PrP^{Sc} and infectivity in the lymphoreticular tissues.

From the perspective of pre-clinical diagnosis, the sensitivity of diagnostic methods and the procedures to concentrate PrP^{Sc} become crucial because the amount of PrP^{Sc} outside the CNS might be extremely small. The detection limit of currently available PrP^{Sc} detection methods, such as ELISA , is about 2 pM (Ingrosso, L., Vetrugno, V., Cardone, F. and Pocchiari, M. (2002) Molecular diagnostics of transmissible spongiform encephalopathies. *Trends in Molecular Medicine* 8*,* 273-280). An improved extractions method for PrP^{Sc} with sodium phosphotungstate (Wadsworth, J.D.F. et al. (2001) Tissue distribution of protease resistant prion protein in variant Creutzfeldt-Jakob disease using a highly sensitive immunoblotting assay. *Lancet* 358, 171-180) and newly discovered molecules, such as plasminogen (Fischer, M.B., Roeckl, C., Parizek, P., Schwarz, H.P. and Aguzzi, A. (2000) Binding of disease-associated prion protein to plasminogen. *Nature* 408, 479-483) and protocadherin-2 (Brown, P., Cervenakova, L. and Diringer, H. (2001) Blood infectivity and the prospects for a diagnostic screening test in Creutzfeldt-Jakob disease. *J Lab Clin Med* 137, 5-13) binding with high affinity to PrP^{Sc}, might boost new hopes for preclinical diagnostics of TSE. An original approach to increase the minimum detectable level of PrP^{Sc} comes from Saborio and co-worker (Saborio, G.P., Permanne, B. and Soto, C. (2001) Sensitive detection of pathological prion protein by cyclic amplification of protein misfolding. *Nature* 411, 810-813), who developed an efficient protocol for the 10-100-fold amplification of PrP^{Sc}.

### OBJECTS AND SUMMARY OF THE INVENTION

It is therefore an object of the invention to provide an alternative possibility to reversibly aggregate/dissociate polypeptides. It is also an object of the invention to provide ligands that selectively bind to the three-dimensional structure of such polypeptide aggregates. It is a further object of the present invention to provide sensors, which detect binding of ligands to the three-dimensional structures of such polypeptide aggregates.

These and further objects are achieved by the method according to claim 1 and by the proteins according to claim 10. Additional and preferred features derive from the dependent claims.

Structural studies of mammalian prion protein at pH values between 4.5 and 5.5 established that the N-terminal 100-residue domain is flexibly disordered, i.e., has random coil information. This invention describes that at pH values between 6.5 and 7.8, i.e., the pH at the cell membrane, the octapeptide repeats in recombinant human prion protein hPrP(23-230) encompassing the highly conserved sequence PHGGGWGQ are structured. The nuclear magnetic resonance (NMR) solution structure of the OPR at pH 6.2 reveals a new structural motif that causes a reversible pH-dependent PrP oligomerization into macromolecular aggregates. Comparison with the crystal structure of HGGGW-Cu²⁺ indicates that the binding of copper ions induces a conformational transition that presumably modulates PrP aggregation. These results suggest a functional role of the cellular prion protein in homophylic cell adhesion within the synaptic cleft.

Bioreceptors can provide the basis for specific and sensitive biosensors (Schultz, JS. (1987) Sensitivity and dynamics of bioreceptor-based biosensors. Ann. N Y Acad. Sci. 506, 406-414). There are many sources of bioreceptors in nature for biochemicals of interest in biotechnology and biomedicine. These bioreceptors (antibodies, enzymes, membrane proteins, binding proteins) can be modified and produced in large quantities using modern biotechnological techniques (Rigler, P., Ulrich, W.P., Hoffmann, P., Mayer, M. and Vogel, H. (2003) Reversible immobilization of peptides: surface modification and in situ detection by attenuated total reflection FTIR spectroscopy. Chemphyschem. 4, 268-275). The characteristics of the biosensor can also be fine-tuned by modifying the structure of the analog-analyte, which will also provide several orders of magnitude of range sensitivity with a given bioreceptor. The ultimate sensitivity of the biosensor may be limited by the dissociation kinetics of the reaction between analyte and bioreceptor because there is a trade-off between sensitivity and sensor response rate to changes in analyte concentration.

Advantages of the present invention comprise:
• Polypeptides or proteins with inherent aggregation capability can be oligomerized by the presence and the structure of peptide repeats localized in a domain of this polypeptide which is flexibly disordered dependent of the pH.
• The peptide repeats required for reversible polypeptide aggregation/dissociation may be - alone or together with a flexibly disordered protein domain - part of the native amino acid sequence or may be introduced by posttranslational modification of a polypeptide or protein.
   o Oligomerization only is dependent on the pH of the fluidic environment of the polypeptides or proteins.

### BRIEF DESCRIPTION OF THE FIGURES

The following figures are intended to document prior art as well as the invention. Preferred embodiments of the method in accordance with the invention will also be explained by means of the figures, without this being intended to limit the scope of the invention.
- Fig. 1.: Primary structure of the human prion protein;
- Fig. 2.: Apparent molecular weight of hPrP polypeptides;
- Fig. 3.: pD dependence of hPrP ¹H NMR spectrum:
Fig. 3A hPrP(23-230);
Fig. 3B hPrP(81-230);
Fig. 3C hPrP(90-230);
- Fig. 4.: Stereo views of octapeptide repeat structures:
Fig. 4A 20 energy-refined DYANA conformers of HGGGWGQP;
Fig. 4B Space-filling model of (HGGGWGQP)₃;
Fig. 4C Comparison of HGGGWGQ and the X-ray structure of HGGGW-Cu²⁺;
- Fig. 5.: Backbone mobility of hPrP(23-230).

### DETAILED DESCRIPTION OF THE INVENTION

NMR solution structures have been described for recombinant forms of intact human (Zahn, R. et al. (2000) NMR solution structure of the human prion protein. *Proc Natl Acad Sci U S A* 97, 145-150), bovine, and murine PrP at pH 4.5, and of the Syrian hamster PrP at pH 5.5. Under acidic conditions, all prion proteins contain a C-terminal globular domain that extends approximately from residues 121-230 containing a two-stranded anti-parallel β-sheet and three α-helices, and an N-terminal domain encompassing residues 23-120 that is flexibly disordered (Figure 1). At pH 7.3, the average interstitial milieu of the brain, there is no detailed structural information available, except for the NMR structure of a C-terminal fragment corresponding to the globular domain of human prion protein, hPrP(121-230), determined at pH 7 (Luigi Calzolai and R.Z., unpublished results) that is largely similar to the structure at acidic conditions (Zahn et al., 2000). In the crystal structure of dimeric hPrP(90-231) that has been recently determined from crystals grown in pH 8 solution, the two globular domains are linked through interchain disulfide bonds.

In an attempt to investigate possible effects of pH on the structure of PrP^{C} we have studied the recombinant human prion protein (hPrP) in solution using NMR spectroscopy and dynamic light scattering. For these studies we have produced recombinant hPrP(23-230) corresponding to mature PrP^{c} as well as two N-terminally truncated PrP constructs (Figure 1). We find that protonation of the four OPR-histidines results in PrP aggregation. From distance constraint calculations of ¹⁵N-labelled hPrP(23-230) we have calculated the NMR structure of the OPR in pH 6.2 solution. This structure is compared with the recently determined crystal structure of the copper binding octapeptide repeat segment HGGGW-Cu²⁺ (Burns, C.S. et al. (2002) Molecular features of the copper binding sites in the octarepeat domain of the prion protein. *Biochemistry* 41, 3991-4001). The results are evaluated with regard to possible functional roles of the OPR in PrP^{C} in the presence and absence of copper.

Furthermore, this invention includes the following applications:
o The provision and application of a new kind of screening test as well as accurate diagnostic methods at early stages of clinical signs or during the pre-clinical phase of TSE (Transmissible Spongiform Encephalopathies), in particular vCJD (new variant of Creutzfeldt-Jakob disease).
   • The immobilization of OPR-tagged fusion proteins or polypeptides to a solid phase, such as resins, glass beads etc., allows a new kind of affinity purification, enrichment or detection of OPR-tagged fusion proteins or polypeptides to be provided and/or applied.
   • The provision of OPR-tagged fusion proteins or polypeptides and their application for a new kind of pH dependent molecular switches for IT technologies, or for molecular sensors or machines working on a molecular level.
   • The provision of OPR-tagged fusion proteins or polypeptides that specifically recognize prion proteins as a therapeutic agent against TSE as well as the provision of gene therapy vectors for the therapy of vCJD.
   • The provision of OPR-tagged fusion proteins or polypeptides for the production of polyclonal, monoclonal, and/or engineered antibodies.
   • The provision of ligands that selectively bind to the three-dimensional structure of such polypeptide aggregates. Such ligands comprise prion proteins (PrP^{C} and PrP^{Sc}), antibodies, chemical molecules, and octapeptide-containing fusion proteins.
   • The provision of OPR-tagged fusion proteins or polypeptides for sensor technology, including chemical (e.g., biochemical) and/or physical (e.g., optical) sensor chips.

### EXPERIMENTAL RESULTS

### 1. Production and Spectroscopic Characterization of Human Prion Proteins:

The following polypeptides were prepared for the present study (Figure 1): the mature form of the human prion protein, hPrP(23-230); hPrP(81-230), containing a single octapeptide; hPrP(90-230), completely lacking the OPR and corresponding approximately to the minimal sequence required for prion propagation; and hPrP(121-230), corresponding to the well-structured globular PrP domain (Zahn et al., 2002). This array of constructs enabled investigations of possible influences of the overall chain length on the solution characteristics of human prion proteins.

### 2. Influence of pH on Hydrodynamic Radius of Human Prion Proteins:

The hydrodynamic radius (R_{H}) of hPrP polypeptides was determined from dynamic light scattering measurements as summarized in Figure 2.

Figure 2 shows the apparent molecular weight of hPrP polypeptides. Dynamic light scattering measurements were carried out at 20 °C with 4 mg/ml protein solutions buffered with 10 mM sodium acetate at pH 4.5 (light gray bars), 10 mM sodium phosphate at pH 7.0 (dark gray bars), or 10 mM sodium acetate at pH 4.5 and containing 100 mM sodium chloride (black bars). Standard errors are given for 4 independent measurements with 30 data points each. The arrow indicates that the apparent molecular weight of hPrP(23-230) at pH 7.0 is larger than 4 MDa.

At pH 4.5, R_{H} of hPrP(121-230) is in good agreement with the molecular size of the monomeric protein. When assuming a spherical globular shape for the C-terminal domain, the estimated apparent molecular weight of hPrP(121-230) is 15.1 kDa compared to 13.1 kDa as calculated from the amino acid sequence. The N-terminal domain of residues 23-120 only slightly reduced the diffusion rate of hPrP molecules in pH 4.5 solution, but, in the presence of 100 mM sodium chloride there was an increase in R_{H} with increasing length of the N-terminus. The effect of salt on apparent molecular weight is rather unspecific as it does not depend on a specific sequence motif.

At pH 7.0, however, immediate precipitation of hPrP(23-230) upon adjusting the protein solution from pH 4.5 to pH 7 precluded an estimation of R_{H} using dynamic light scattering (Figure 2), indicating that the particle size of hPrP aggregates was > 4 MDa. Size exclusion chromatography experiments failed to identify the molecular size of PrP aggregates more exactly because the protein interacted with the agarose-dextran gel, presumably owing to an affinity of the OPR for the polysaccharide (Hundt, C. et al. (2001) Identification of interaction domains of the prion protein with its 37-kDa/67-kDa laminin receptor. *Embo Journal* 20, 5876-5886). In contrast, the C-terminal fragments hPrP(81-230), hPrP(90-230) and hPrP(121-230) only showed a slight increase in R_{H} when compared to the measurements in pH 4.5 solution at low ionic strength, indicating that the highly specific aggregation of hPrP(23-230) into macromolecular protein particles can be attributed to the N-terminal segment of residues 23 to 89 encompassing the OPR (Figure 1).

### 3. Influence of pD on ¹H NMR Linewidth of Human Prion Proteins:

To further characterize the pH-dependent aggregation of hPrP(23-230) we performed ¹H NMR experiments at various solution conditions. ¹H linewidths in NMR experiments are approximately proportional to the overall rotational correlation time (τ_{c}) and thus depends on the molecular mass and shape of the molecule.

Linewidths significantly larger than expected based on the molecular mass of a protein imply either an increase in τ_{c} due to aggregation or that chemical exchange or conformational exchange effects contribute significantly to the linewidth.

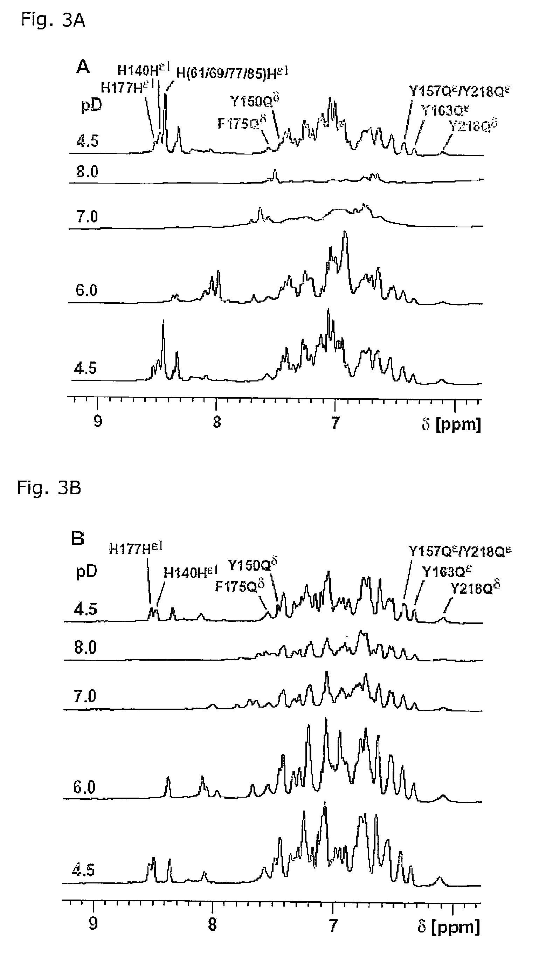

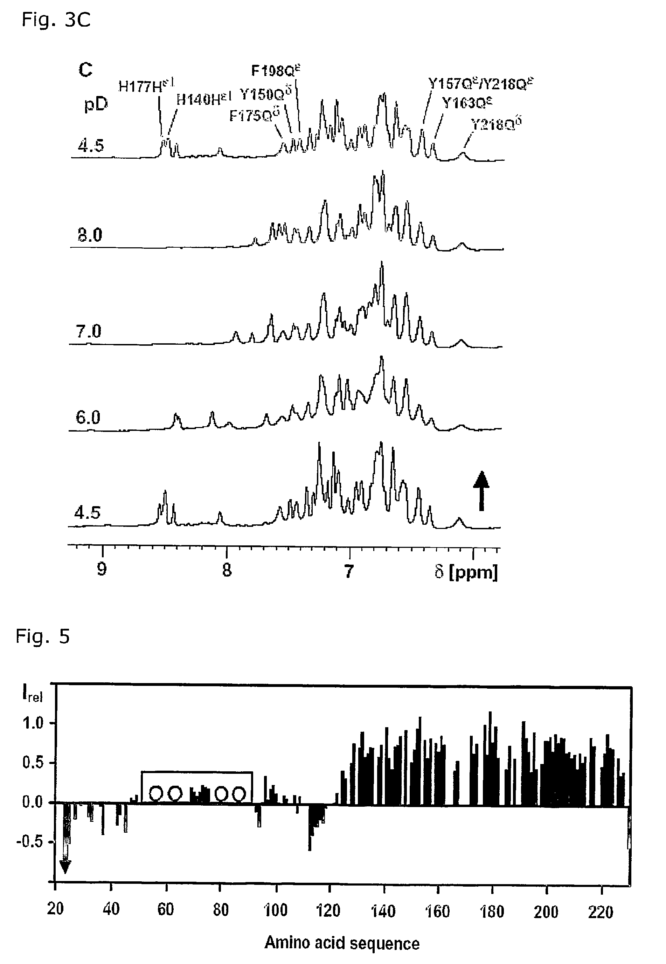

Figure 3 shows the pD dependence of hPrP ¹H NMR spectrum. Shown is the spectral region from 6 to 9 ppm in the 750 MHz ¹H NMR spectrum of a 0.6 mM solution of hPrP in D₂O at 20 °C. (A) hPrP(23-230). (B) hPrP(81-230). (C) hPrP(90-230). Prior to these experiments, the labile protons were exchanged with deuterons by dissolving samples in D₂O. Subsequently, the pD of the sample was increased in a stepwise fashion (see arrow bottom to top) by adding small amount of NaOD, before decreasing it again to pD 4.5 by small additions of DCI. Resonance assignments for selected aromatic resonance signals are indicated at the top of each spectrum.

Figure 3A shows the spectral region from 6 to 9 ppm in the ¹H NMR spectrum of hPrP(23-230) recorded in D₂O. At pD 4.5, the aromatic ring protons of His, Phe and Tyr residues located within the folded C-terminal domain show linewidths typical for a globular protein of about 23 kDa. The less dispersed resonance lines of the flexibly disordered tail such as the overlapping resonances of H^{ε1} of histidines 61, 69, 77 and 85 are significantly narrower because their effective τ_{c} is smaller due to the increased mobility in the tail. As the pD was increased stepwise from 4.5 to 8, the H^{ε1} resonances of His shifted up-field and the ¹H linewidths generally increases, as shown for the aromatic ring protons in Figure 3A. The changes were reversible as indicated by the top spectrum. Repeating the same experiment with hPrP(81-230) resulted in a slight line broadening of resonance signals (Figure 3B), whereas for hPrP(90-230) the linewidth was independent on pD (Figure 3C).

As these measurements were performed in D₂O where only non-exchangeable protons are detected, we can rule out chemical exchange as a possible source for the observed uniform line broadening in the NMR spectra. Furthermore, an exclusive effect of conformational exchange on linewidth would be considerably smaller than is observed in Figure 3A, and the recorded NMR spectra do not resemble that of a molten globule protein with poorly dispersed resonances (Dyson, H.J. and Wright, P.E. (2001) Nuclear magnetic resonance methods for elucidation of structure and dynamics in disordered states. *Nuclear Magnetic Resonance of Biological Macromolecules, Pt B* 339, 258-270). More likely, and in accordance with the dynamic light scattering experiments (Figure 2), the progressive broadening of NMR peaks at pD values between 6.0 and 8.0 is caused by protein aggregation owing to the deprotonation of His side chains within the peptide segment 23-89, i.e. the four OPR-histidines (Figure 1), resulting in the observed up-field shift of H^{ε1} resonances (Figure 3A). There was no line broadening in [¹⁵N,¹H]-correlation spectroscopy (COSY) spectra recorded with equimolar mixtures of unlabelled hPrP(23-230) and ¹⁵N-labelled hPrP(121-230) in H₂O solution at pH 7.0 (data not shown), indicating that the binding epitope of the OPR is located within the N-terminal segment 23-120.

### 4. Resonance Assignment of the N-terminal Domain at pH 6.2:

Sequence specific assignments of backbone amide protons and nitrogens of the N-terminal segment 23-120 at pH 6.2 was obtained from [¹⁵N,¹H]-COSY pH-titration experiments with ¹⁵N-Iabeled hPrP(23-230) based on chemical shift comparison with spectra recorded at pH 4.5 (Liu, A.Z., Riek, R., Wider, G., von Schroetter, C., Zahn, R. and Wüthrich, K. (2000) NMR experiments for resonance assignments of C-13, N-15 doubly-labeled flexible polypeptides: Application to the human prion protein hPrP(23-230). *Journal of Biomolecular Nmr* 16, 127-138). At pH 6.2, where about 40% of the unperturbed histidines are deprotonated, the resonance lines in the [¹⁵N,¹H]-COSY spectrum are only slightly broadened, indicating that a large fraction of PrP molecules is monomeric under these conditions. The backbone assignments were confirmed using a three-dimensional ¹⁵N-resolved [¹H,¹H]-nuclear Overhauser enhancement spectroscopy (NOESY) spectrum, which was subsequently used for assignment of side chain protons. All polypeptide backbone resonances were assigned, excluding the amide nitrogens and amide protons of Gly35, Gly93 and Gly94, which are overlapped with those of Gly residues in the OPR region (Liu et al., 2000). Corresponding resonance lines of the individual OPR segments overlap completely, except for the two flanking dipeptides Gln52-Gly53 and Gly90-Gln91 (Figure 1), i.e. the resonance of a given atom from a given residue in the octapeptide occur at the same frequency for all five repeats. Among the labile side chain protons, the amide groups of all 4 Asn and 8 Gln residues could be assigned using intrare-sidual NOEs, with the sole exception of Gln59. The ε-proton resonances of the 3 Arg residues could not be detected at pH 6.2 due to fast exchange with the solvent.

### 5. Collection of Conformational Constraints and Structure Calculations of the N-terminal Domain at pH 6.2:

For assignment of NOESY cross peaks we used the automatic NOE assignment software CANDID in combination with the structure calculation program DYANA. At the outset of the structure calculation of the peptide segment 23-120 in hPrP(23-230), a total of 689 NOESY cross peaks were assigned and integrated in the ¹⁵N-resolved [¹H,¹H]-NOESY spectrum recorded at pH 6.2, which yielded 322 NOE upper limit distance constrains. Strikingly, a total of 219 NOESY cross peaks could be identified as originating from amide nitrogens and protons of the OPR region at pH 6.2, whereas only 98 such peaks are observed at pH 4.5 (Liu et al., 2002). This indicated the presence of additional structured regions in the OPR at pH 6.2, which are not stable at pH 4.5. In contrast, no additional NOESY cross peaks could be identified for residues outside the OPR, indicating that the pH-dependent structure formation is limited to this region.

Every cross peak within the ¹⁵N-resolved [¹H,¹H]-NOESY could be the result of an interaction of an amide proton with a second proton of the same octapeptide or with one of the other octapeptides. To investigate the compatibility of the NOESY cross peaks with intra- and inter-octapeptide assignments, we performed a structure calculation of a 16-residue peptide (PHGGGWGQ)₂ corresponding to two OPR using the programs CANDID and DYANA, where the same chemical shifts were attributed to corresponding atoms of a given residue within the two OPR. Out of the resulting 80 NOE upper distance constraints 11 constraints were assigned as inter-octapeptide involving the C-terminal Gln of the first octapeptide: 7 sequential (i,i+1) NOEs with Pro, 2 medium-range (i,i+2) NOEs with His, and 2 long-range (i,i+5) NOEs with Trp.

To further improve the structure calculation of OPR we investigated the compatibility of the NOESY cross peaks with various possible assignments within a single octapepide. We performed a series of CANDID / DYANA structure calculations using the same peak list as an input, except that the amino acid sequence within the chemical shift list was varied with respect to the standard OPR sequence PHGGGWGQ (Figure 1). The results in Table 1 show that all eight structure calculations converged with a residual DYANA target function value close to 1.

**Table 1: Characterization of OPR Calculated after NOE Assignment with CANDID and DYANA^{a}**

| | | Target Function^{c} | | | |
|---|---|---|---|---|---|
| Sequence | NOEs^{b} | 1 OPR | 2 OPR | 3 OPR | 4 OPR |
| PHGGGWGQ | 110 | 0.42 ± 0.05 | 2.09 ± 0.85 | 5.14 ± 1.32 | 5.16 ± 1.68 |
| HGGGWGQP | 98 | 0.28 ± 0.02 | 0.62 ± 0.07 | 1.12 ± 0.19 | 1.18 ± 0.17 |
| GGGWGQPH | 93 | 0.55 ± 0.03 | 1.11 ± 0.04 | 2.40 ± 0.91 | 4.64 ± 1.66 |
| GGWGQPHG | 96 | 0.66 ± 0.58 | 3.66 ± 1.93 | 6.39 ± 2.59 | 11.1 ± 3.58 |
| GWGQPHGG | 96 | 0.05 ± 0.01 | 4.65 ± 1.36 | 5.72 ± 3.90 | 9.78 ± 6.03 |
| WGQPHGGG | 98 | 0.97 ± 0.12 | 2.75 ± 0.65 | 5.67 ± 1.22 | 9.91 ± 1.89 |
| GQPHGGGW | 102 | 1.11 ± 0.29 | 2.90 ± 0.84 | 7.15 ± 1.96 | 11.4 ± 3.03 |
| QPHGGGWG | 102 | 1.13 ± 0.12 | 2.63 ± 0.24 | 5.58 ± 1.06 | 9.51 ± 2.35 |

| | | | | | |
|---|---|---|---|---|---|
| Captions to Table 1: ^{a} Eight different variant octapeptide sequences were generated by starting at different sequence positions within the OPR region of residues 60-91 (Figure 1). The input for the DYANA calculations with segments of two, three and four OPR was adapted from the seventh cycle of the CANDID / DYANA output of a single OPR the sequence of which is listed in the first column. Each calculation was started with 100 randomized structures. ^{b} Number of NOE upper distance constraints in the input of each OPR. ^{c} Residual DYANA target function value (Å²). The average ± standard deviation is given for a bundle of 20 conformers used to represent the structure. | | | | | |

However, when the DYANA calculations were repeated using the same distance constraints but for peptides containing two, three or four consecutive OPR, the resulting structures converged with different target function values. Constantly small residual constraint violations were only obtained for those peptides with the repetitive sequence element HGGGWGQP (Table 1), indicating that these structures are mostly consistent with the experimental constraints and are thus sterically more favorable than the structures of the other seven octapeptide sequences.

The 20 best DYANA conformers used to represent the NMR structure of the 8-residue peptide HGGGWGQP and the 24-residue peptide (HGGGWGQP)₃, corresponding to residues 61 to 84 of hPrP(23-230) (Figure 1), were further energy-refined with the program OPALp. Table 2 gives a survey of the results of the structure calculation.

**Table 2: Characterization of the 20 Energy-refined DYANA Conformers Representing the NMR Structures of HGGGWGQP and (HGGGWGQP)₃**

| Quantity | Value¹ | |
|---|---|---|
| | HGGGWGQP | (HGGGWGQP)₃ |
| Residual DYANA target function value (Å²)² | 0.28 ± 0.02 | 1.12 ± 0.19 |

| Residual NOE distance constraint violations | | |
|---|---|---|
| Number > 0.1 Å | 1 ± 1 | 2 ± 2 |
| Maximum (Å) | 0.10 ± 0.01 | 0.11 ± 0.00 |

| AMBER energy (kcal/mol) | | |
|---|---|---|
| Total | -6819 ± 341 | -6850 ± 200 |
| van der Waals | +13 ± 18 | -7 ± 22 |
| Electrostatic | -8569 ± 324 | -8589 ± 204 |

| RMSD from ideal geometry | | |
|---|---|---|
| Bond lengths (Å) | 0.0078 ± 0.0001 | 0.0079 ± 0.0001 |
| Bond angles (degrees) | 1.88 ± 0.03 | 1.91 ± 0.03 |
| RMSD, N, C°, C' (Å)³ | 0.26 ± 0.05 | 3.19 ± 0.80 |
| RMSD, all heavy atoms (Å)³ | 0.62 ± 0.09 | 3.52 ± 0.88 |

| | | |
|---|---|---|
| Captions to Table 2: ¹ Average values ± standard deviations for the 20 energy-minimized conformers with the lowest DYANA target function values are given. The input consisted of 98 and 294 NOE upper distance constraints for HGGGWGQP and (HGGGWGQP)₃, respectively. ² Before energy minimization. ³ RMSD values relative to the mean coordinates. | | |

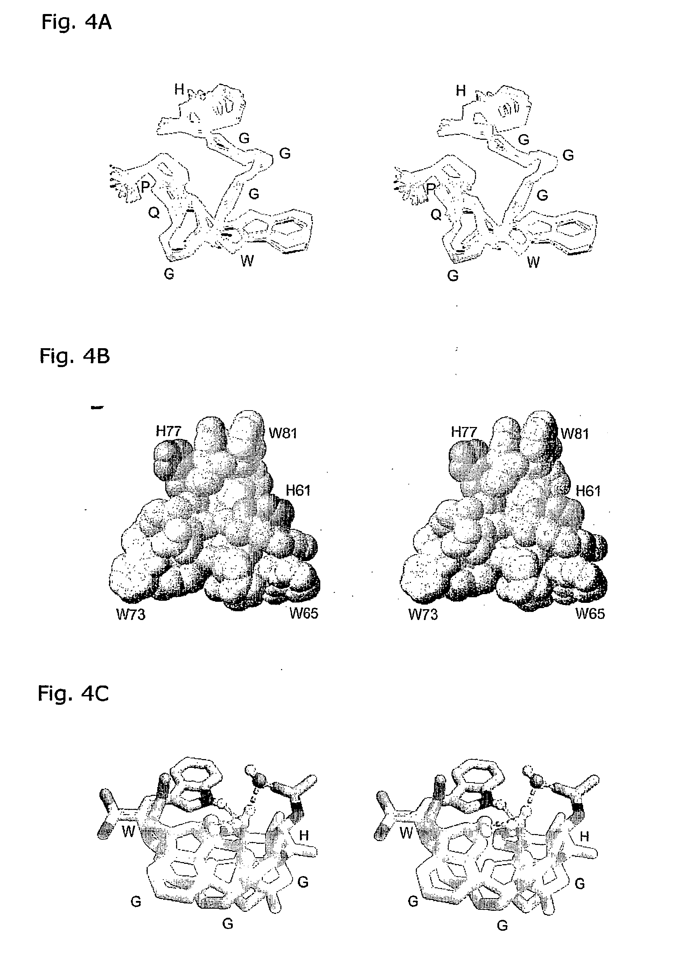

Table 2 shows that the global RMSD values among the bundle of 20 conformers of HGGGWGQP are representative of a high-quality structure determination (Figure 4A), whereas the NMR structure of (HGGGWGQP)₃ is less precisely defined because of the missing assignments of long-range NOEs connecting the OPR.

Figure 4 shows stereo views of octapeptide repeat structures. (A) All-heavy-atom representation of the 20 energy-refined DYANA conformers superimposed for best fit of the N, C° and C' atoms of HGGGWGQP. The backbone is gray and the side chains are shown in different colors: Trp (yellow), His (cyan), Gln (pink) and Pro (orange). (B) Space-filling model of (HGGGWGQP)₃. The numbering corresponds to residues 61 to 84 in the human prion protein sequence (Figure 1). The same color code as in (A) was used. (C) Comparison of the NMR structure of HGGGWGQP and the X-ray structure of HGGGW-Cu²⁺ (Burns et al., 2002). The relative orientation of the two molecules resulted from a superposition for best fit of the backbone heavy atoms of the pentapeptide segment HGGGW (RMSD 1.3 Å). The backbone and side chain heavy atoms of the NMR structure are in green. In the X-ray structure the oxygen, nitrogen, carbon and hydrogen atoms are displayed in red, blue, gray and white, respectively. Hydrogen bonds between the pentapeptide and ordered water molecules are indicated as white dashed lines. The position of the copper ion is indicated by a sphere in cyan (for illustrative purposes, the copper radius is not scaled to reflect the true atomic radius). The red and blue lines indicate the copper coordination sites between copper and the peptide oxygen and nitrogen atoms, respectively.

### 6. NMR Structure of Octapeptide Repeats (HGGGWGQP) and (HGGGWGQP)₃:

The NMR structure of HGGGWGQP has the same global fold as the corresponding OPR in (HGGGWGQP)₃, with an RMSD value of 0.32 Å between the backbone heavy atoms in the mean structures of HGGGWGQP and the N-terminal octapeptide of (HGGGWGQP)₃ corresponding to residues 61-68 of hPrP(23-230). The segments HGGGW and GWGQ adopt a loop conformation and a β-turn structure, respectively, where the β-turn is corroborated by a continuous pattern of *d*_{NN} and *d*_{aN} NOE connectivities, and a *d*_{aN}(i,i+2) NOE connectivity between Trp and Gln. In (HGGGWGQP)₃ the octapeptides are arranged to form a triangular globular domain (Figure 4B). This molecular architecture is stabilized by a repetitive set of hydrogen bonds: each of the three OPR contains three intra-octapeptide hydrogen bonds His(*i*) H^{N}-O' Gln(*i*+*6*), Gly(*i*+*2*) H^{N}-N^{ε2} His(*i*) and Trp(*i*+*3*) H^{ε}-O' Gly(*i*). The contacts between the three OPR are stabilized by two hydrogen bonds of the type Gly(*i*+*2*) H^{N}-O' Pro(*i*). The peptide bonds of Gln(*i*)-Pro(*i*+*1*) are in *trans* conformation. All side chain atoms are largely solvent exposed including the hydrophobic side chains of Trp (Figure 4B). From the three-dimensional structure of the OPR it thus appears likely that the symmetric distribution of solvent exposed hydrophobic residues is of importance for PrP aggregation.

### 7. Backbone Dynamics of hPrP(23-230) at pH 6.2:

The formation of tertiary structure interactions at pH 6.2 within the OPR correlates with intramolecular rate processes that may be detected by measurement of heteronuclear ¹⁵N{¹H}-NOEs. In previous studies in pH 4.5 solution (Zahn et al., 2002) the N-terminal domain comprising residues 23-120 of hPrP(23-230) showed exclusively negative NOEs, contrasting with the C-terminal domain which displayed values typical for a globular structured domain. Thus, the effective rotational correlation times, τ_{c}, could be estimated to be at least several nanoseconds for the backbone ¹⁵N-¹H moieties of the *C*-terminal domain, whereas the ¹⁵N-¹H moieties in the N-terminal domain must have τ_{c} < 1 ns as would be expected for a flexible random coil-like polypeptide chain. In contrast, at pH 6.2 some of the ¹⁵N-¹H moieties of the N-terminal domain, including the OPR and several residues flanking the OPR (Figure 5), show positive ¹⁵N{¹H}-NOEs of about 0.2 indicating that this polypeptide region is folded into a globular structure with a certain degree of mobility, presumably because it is in equilibrium with more unfolded conformations.

Figure 5 shows the backbone mobility of hPrP(23-230). Steady-state ¹⁵N{¹H}-NOEs of amide groups were measured in a 0.5 mM solution of hPrP(23-230) in 90% H₂O/10% D₂O at pH 6.2 and 20 °C. In the box from positions 51 to 91 the circles indicate that patterns are identical for all five repeats due to the degenerate chemical shifts (see text). The arrow indicates that the ¹⁵N{¹H}-NOEs are lower than -1 for Lys23 and Lys24. Some of the ¹⁵N{¹H}-NOEs could not be quantified because of spectral overlap.

In addition to the backbone amide groups the Trp indole ¹⁵N^{ε}-¹H moieties of the OPR were characterized by NOE values close to zero, implying that the side chains may be involved in transient tertiary structure interactions, in agreement with the results from the structure calculations. Although the aggregation of hPrP(23-230) at pH values higher than 6.2 precluded a detailed NMR characterization under these conditions, it appears likely that the globular structure of OPR is further stabilized at pH 7 because of the increased degree of deprotonation of the histidines.

### DISCUSSION OF THE RESULTS

### 1. Octapeptide Repeat Structure Represents a New Structural Motif:

The program DALI (Holm, L. and Sander, C. (1993) Protein-Structure Comparison by Alignment of Distance Matrices. *Journal of Molecular Biology* 233, 123-138) revealed no significant similarity between the structures of HGGGWGQP and (HGGGWGQP)₃ described here with any of the previous deposits in the Protein Data Bank, indicating that the OPR structure represents a new structural motive. The results of our structure calculations deviate from previous structural studies on synthetic OPR peptides. From circular dichroism experiments at pH 7.4 it was suggested that the OPR adopt an extended conformation with properties similar to a poly-L-proline type II helix (Smith, C.J., Drake, A.F., Banfield, B.A., Bloomberg, G.B., Palmer, M.S., Clarke, A.R. and Collinge, J. (1997) Conformational properties of the prion octa-repeat and hydrophobic sequences. *Febs Letters* 405, 378-384), whereas a recent NMR study carried out between pH 6.2 and 6.6 suggests that the segments HGGGW and GWGQ adopt a loop conformation and a β-turn, respectively (Yoshida, H., Matsushima, N., Kumaki, Y., Nakata, M. and Hikichi, K. (2000) NMR studies of model peptides of PHGGGWGQ repeats within the N-terminus of prion proteins: A loop conformation with histidine and tryptophan in close proximity. *Journal of Biochemistry* 128, 271-281). Although we also observe a turn-like conformation for segment GWGQ (Figure 4A), the loop conformation in our structure is different because a close proximity of the imidazole side chain of His to the aromatic ring of Trp is not supported from our structure calculation. Because NMR data on cyclized OPR encompassing one or two octapeptides also do not suggest a close proximity of His and Trp side chains (Yoshida et al., 2000) this interaction might only transiently be formed.

Variants of mammal octapeptides comprise sequences, such as PHGGSWGQ (mouse) and PHGGGWSQ (rat) or pseudooctapeptides, e.g. deriving from these octapeptides, with more or less than eight amino acids, such as PHGGGGWSQ (various species) or PHGGGSNWGQ (marsupial). Non-mammal hexapeptides comprise sequences, such as PHNPGY (chicken) or PHNPSY, PHNPGY (turtle) or pseudohexapeptides, e.g. deriving from these hexapeptides, with more or less than six amino acids. The sequences discussed here are to be understood as examples that do not limit the gist of this invention.

### 2. Possible Role of Copper in Modulation of pH-dependent PrP Aggregation:

Unexpectedly, the HGGGW loop in the NMR structure of HGGGWGQP has a similar backbone fold as the corresponding resides in the crystal structure of the copper binding octapeptide repeat segment HGGGW-Cu²⁺ recently determined from crystals grown at pH 7.4 (Burns, C.S. et al. (2002) Molecular features of the copper binding sites in the octarepeat domain of the prion protein. *Biochemistry 41,* 3991-4001). In the structure of HGGGW-Cu²⁺ (Figure 4C), Cu²⁺ is pentacoordinated with equatorial ligation from the δ1-nitrogen of the His imidazole and the amide nitrogens from the next two Gly residues of which the second Gly also contributes its amide carbonyl oxygen. With the exception of the His backbone nitrogen and C°, all atoms from the His through the nitrogen of the third Gly lie approximately in the equatorial plane and the copper is just above this plane, consistent with a pentacoordinate complex. The Trp indole also participitates through a hydrogen bond to the axially coordinated water molecule, whereas glutamine is the only side chain possessing a functional group that does not participitate in copper binding. From their data Burns and co-workers suggested a model where exposed glutamine side chains within two "metal sandwich" octapeptide repeats of membrane bound PrP may serve as an interaction site for intermolecular recognition between PrP molecules, and thus stimulating copper induced endocytosis (Pauly, P.C. and Harris, D.A. (1998) Copper stimulates endocytosis of the prion protein. J *Biol Chem* 273, 33107-33110) or facilitating the formation of PrP^{Sc}.

Although the NMR structure of the copper-free HGGGW-loop has a similar backbone conformation to the corresponding residues in HGGGW-Cu²⁺, with an RMSD value of 1.3 Å between the backbone heavy atoms of the two pentapeptides (Figure 4C), there are obvious conformational differences for the aromatic side chains involved in cooper coordination in the HGGGW-Cu²⁺ structure. In the copper-free HGGGW the His imidazole shifts below and tilts towards the equatorial plane of the copper pentacoordinate complex in the HGGGW-Cu²⁺ structure, resulting in an increase of the distance between the δ1-nitrogen of His and the Cu²⁺ binding site from 1.9 Å to 3.5 Å. Furthermore, the Trp indole in HGGGW is flipped by about 180° around a virtual axis parallel to one passing through the coordinating nitrogen and Cu²⁺, thus precluding the formation of a hydrogen bond between εNH of Trp and the oxygen atom of the axial water molecule observed in the HGGGW-Cu²⁺ structure.

From the combination of the structural and biochemical data reported here and in previous publications (Aronoff-Spencer, E. et al. (2000) Identification of the Cu²⁺ binding sites in the N-terminal domain of the prion protein by EPR and CD spectroscopy. *Biochemistry* 39, 13760-13771; Viles, J.H., Cohen, F.E., Prusiner, S.B., Goodin, D.B., Wright, P.E. and Dyson, H.J. (1999) Copper binding to the prion protein: structural implications of four identical cooperative binding sites. *Proc Natl Acad Sci U S A* 96, 2042-2047) the conformation of the HGGGW-loop within the OPR appears to depend on both pH *and* copper binding:

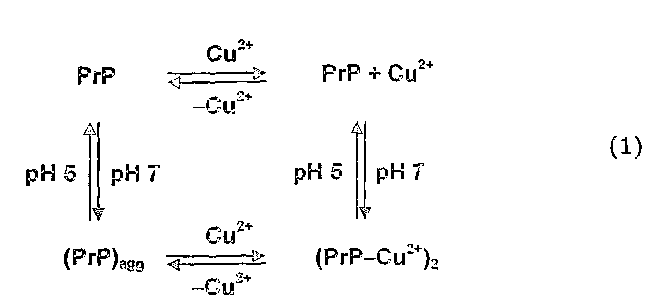

According to scheme (1), at pH values between 4.7 and 5.8, i.e. the pH of endosome-like compartments, the OPR-histidines are largely protonated: consequently, the OPR are flexibly disordered and bind copper only with low affinity and cooperativity. At pH values between 6.5 and 7.8, i.e. the pH at the cell membrane, the OPR-histidines are predominantly deprotonated, thus stabilizing the HGGGW-loop conformation which promotes aggregation, and if present, Cu²⁺ is incorporated into the copper binding sites. The coordination of copper by HGGGW results in a slight but significant conformational change that presumably leads to a structural change in PrP aggregates. The function of copper could thus be that of a modulator of pH-dependent PrP aggregation, although it remains to be shown if the binding of Cu²⁺ is compatible with a reverse aggregation of the OPR into dimeric or oligomeric protein aggregates.

It was not known in the prior art that PrP^{c} forms large protein aggregates. In addition, the finding that aggregation of PrP^{c} is dependent on the pH of the fluidic environment is new. Moreover, it was not known that the OPR are responsible for the pH dependent aggregation of PrP^{c} and that a conformational change is involved in the pH dependence of the aggregation of this OPR. Present database are void of 3D structures similar to that reported in Fig. 4. The oligomerization reaction depends on the pH of the fluidic environment and oligomerization occurs also in absence of monovalent or divalent cations, such as Hg²⁺, Ni²⁺, Sn²⁺ or Cu²⁺ ions.

### 3. Implications of pH-dependent Aggregation on PrP^{C} Physiological Function:

Assuming that natural PrP^{C} behaves similarly *in vivo* as compared to the recombinant hPrP, its aggregation state may also largely depend on the environmental pH. The His containing OPR could therefore act as a pH-dependent aggregation site that concentrates a large number of PrP^{c} molecules within the lipid rafts of the presynaptic membrane surface. A lipid raft of 44 nm diameter, would provide enough surface for about 80 PrP^{C} molecules with a diameter of 5 nm. Thus, the physiological role of copper could be to modulate the number of PrP^{C} molecules within the lipid rafts, thereby stimulating PrP^{C} endocytosis into presynaptic vesicles, where the prion proteins would dissociate into monomers because of the locally acidic pH. This model would be in line with a proposal of Burns and co-workers (Burns et al., 2002), except that copper acts as a modulator rather than an inducer of PrP^{C} aggregation.

Alternatively, the OPR in mammalian PrP^{C} may serve as an intercellular contact site for cell-cell adhesion between neuronal axons and dendrites. A potential involvement of PrP^{C} in cell adhesion has recently been demonstrated by Lehmann and colleagues (Mange, A., Milhavet, O., Umlauf, D., Harris, D. and Lehmann, S. (2002) PrP-dependent cell adhesion in N2a neuroblastoma cells. *Febs Letters* 514, 159-162). They showed that neuroblastoma cells overexpressing PrP^{c} exhibit an increased aggregation behavior when compared to non-transfected cells. Addition of copper chelators or cation chelators during the cell aggregation assay had no significant effect, indicating that PrP^{C}-mediated adhesion occurs in a cation-independent manner. Treatment of neuroblastoma cells with a polyclonal antibody P45-66 that was raised against a synthetic peptide encompassing residues 45-66 of murine PrP (Lehmann, S. and Harris, D.A. (1995) A mutant prion protein displays an aberrant membrane association when expressed in cultured cells. *J Biol Chem* 270, 24589-24597) significantly inhibited cell aggregation. From these results it was concluded that PrP^{C} could function as an adhesion molecule in neuronal cells, with cell aggregation being mediated by specific transcellular binding of PrP^{c} to a heterologous protein such as N-CAM or laminin receptor precursor. However, based on our finding that the OPR constitute a pH-dependent aggregation site, it appears also possible that PrP^{C} is involved in homophilic cell-cell recognition. This is consistent with aggregation suppressing activity of the antibody P45-66 whose epitope comprises the His containing OPR that are responsible for pH-dependent PrP aggregation (Figure 1). The linear combination of two GPI anchored PrP^{c} molecules in adjacent cells interact through an aggregation site in the OPR within an otherwise largely unstructured N-terminal domain would easily span the 20-30 nm distance of the synaptic cleft (Agnati, L.F., Zoli, M., Stromberg, I. and Fuxe, K. (1995) Intercellular Communication in the Brain - Wiring Versus Volume Transmission. *Neuroscience* 69, 711-726). It is thus conceivable that prion proteins are similar to other homophilic cell adhesion molecules such as the cadherins (Pokutta, S. and Weis, W.I. (2002) The cytoplasmic face of cell contact sites. *Current Opinion in Structural Biology* 12, 255-262), which are critically important for establishing brain structure and connectivity during early development. Moreover, PrP^{C} could participate in remodeling synaptic architecture and modifying the strength of the synaptic signal, thus playing an active role in synaptic structure, function, and plasticity. Because the cellular aggregation activity of PrP^{c} does not depend on copper, the role of copper might be that of a chaperone allowing PrP^{c} to switch between two oligomeric conformations with independent cellular functions, i.e. from copper-independent cell-cell adhesion to copper-dependent endocytosis and *vice versa.*

### MATERIALS AND METHODS

### 1. Sample Preparation:

Cloning, expression and purification of hPrP polypeptides in unlabeled form or with uniform ¹⁵N-labeling was achieved as previously described (Zahn, R., von Schroetter, C. and Wüthrich, K. (1997) Human prion proteins expressed in *Escherichia coli* and purified by high-affinity column refolding. *FEBS Lett 417,* 400-404). Protein solutions were concentrated using Ultrafree-15 Centrifugal Filter Devices (Millipore).

### 2. NMR Measurements and Structure Calculations:

The NMR measurements were performed on Bruker DRX500, DRX750 and DRX800 spectrometers equipped with four radio-frequency channels and triple resonance probeheads with shielded z-gradient coils, with unlabeled or ¹⁵N-labeled samples of 1 mM protein solutions in 90% H₂O/10% D₂O or 99.9% D₂O and at 20 °C. For the collection of conformational constraints, a three-dimensional ¹⁵N-resolved [¹H,¹H]-NOESY spectrum in H₂O was recorded at 800 MHz with a mixing time τₘ = 100 ms at T = 20 °C, 207(*t*₁) x 39(*t*₂) x 1024(*t*₃) complex points, *t*_{1,max}(¹H) = 23.0 ms, *t*_{2,max}(¹⁵N) = 21.4 ms, *t*_{3,max}(¹H) = 114 ms, and this data set was zero-filled to 512 x 128 x 2048 points. Processing of the spectra was performed with the program PROSA (Güntert, P., Dotsch, V., Wider, G. and Wüthrich, K. (1992) Processing of Multidimensional Nmr Data with the New Software Prosa. *Journal of Biomolecular Nmr* 2, 619-629). The ¹H and ¹⁵N chemical shifts have been calibrated relative to 2,2-dimethyl-2-silapentane-5-sulfonate, sodium salt.

Steady-state ¹⁵N{¹H}-NOEs were measured at 500 MHz following Farrow *et al.,* (Farrow, N.A., Zhang, O.W., Formankay, J.D. and Kay, L.E. (1994) A Heteronuclear Correlation Experiment for Simultaneous Determination of N-15 Longitudinal Decay and Chemical-Exchange Rates of Systems in Slow Equilibrium. *Journal of Biomolecular Nmr* 4, 727-734) using a proton saturation period of 3 s by applying a cascade of 120-degree pulses in 5 ms intervals; *t*_{1,max}(¹⁵N) = 117.4 ms, *t*_{2,max}(¹H) = 146.3 ms, time domain data size 250(*t*₁) x 1024(*t*₂) complex points.

NOE assignment was obtained using the CANDID software (Herrmann, T., Güntert, P. and Wüthrich, K. (2002) Protein NMR structure determination with automated NOE assignment using the new software CANDID and the torsion angle dynamics algorithm DYANA. *Journal of Molecular Biology* 319, 209-227) in combination with the structure calculation program DYANA (Güntert, P., Mumenthaler, C. and Wüthrich, K. (1997) Torsion angle dynamics for NMR structure calculation with the new program DYANA. *Journal of Molecular Biology* 273, 283-298). CANDID and DYANA perform automated NOE-assignment and distance calibration of NOE intensities, removal of covalently fixed distance constraints, structure calculation with torsion angle dynamics, and automatic NOE upper distance limit violation analysis. As input for CANDID, a peak list of the aforementioned NOESY spectrum was generated by interactive peak picking with the program XEASY (Bartels, C., Xia, T.H., Billeter, M., Güntert, P. and Wüthrich, K. (1995) The Program Xeasy for Computer-Supported Nmr Spectral-Analysis of Biological Macromolecules. *Journal of Biomolecular Nmr* 6, 1-10) and automatic integration of the peak volumes with the program SPSCAN (Ralf Glaser, personal communication). The input for the calculations with CANDID and DYANA contained the NOESY peak list and a chemical shift list from the sequence-specific resonance assignments. The calculation followed the standard protocol of 7 cycles of iterative NOE assignment and structure calculation (Herrmann et al., 2002). During the first six CANDID cycles, ambiguous distance constraints were used. For the final structure calculation, only NOE distance constraints were retained that corresponded to NOE cross peaks with unambiguous assignment after the sixth cycle of calculation. Stereospecific assignments were identified by comparison of upper distance limits with the structure resulting from the sixth CANDID cycle. The 20 conformers with the lowest final DYANA target function values were energy-minimized in a water shell with the program OPALp (Luginbühl, P., Güntert, P., Billeter, M. and Wüthrich, K. (1996) The new program OPAL for molecular dynamics simulations and energy refinements of biological macromolecules. *Journal of Biomolecular Nmr* 8, 136-146), using the AMBER force field. The program MOLMOL (Koradi, R., Billeter, M. and Wüthrich, K. (1996) MOLMOL: A program for display and analysis of macromolecular structures. *Journal of Molecular Graphics* 14, 51-55) was used to analyze the resulting 20 energy-minimized conformers (Tables 1 and 2) and to prepare drawings of the structures.

### 3. Dynamic Light Scattering Experiments:

The dynamic light scattering measurement were performed at 20 °C using a Protein Solutions Ltd. model 801 dynamic light scattering instrument (Hertford, U.K.). The Instrument calculates the translational diffusion coefficient *D*_{T} of the molecules in the sample cell from the autocorrelation function of scattered light intensity data. The hydrodynamic radius *R*_{H} of the scattering particle is derived from *D*_{T}, using the Stokes-Einstein relationship: *D*_{T} = *k*_{B}*T* / 6πη*R*_{H}, where *k*_{B} is the Boltzmann constant, *T* the absolute temperature in Kelvin, η the viscosity of the solvent. Protein concentration was 4 mg/ml in buffer solution containing 10 mM sodium acetate at pH 4.5, or 10 mM sodium phosphate at pH 7.0. Protein solutions were filtered through 100 nm pore-size filters (Whatman, U.K.). To reduce interference with bubbles or dust, 30 data points were analyzed per experiment using the DynaPro dynamic light scattering instrument control software from molecular research DYNAMICS (version 4.0). The hydrodynamic radius *R*_{H} was calculated from the regularization histogram method using the spheres model, from which an apparent molecular weight was estimated according to a standard curve calibrated for known globular proteins.

## Claims

1. A method for the reversible aggregation and/or dissociation of polypeptides, comprising the step of oligomerising a polypeptide at a pH of 6.2 to 7.8 and/or dissociating a polypeptide aggregate at a pH of 4,5 to 5, 5 in a fluid environment, wherein the polypeptide is **characterized in that**
(i) the polypeptide comprises one or more peptide repeat structures derived from prion proteins;
(ii) and that the protein oligomerizes in fluid at a pH of 6,2 to 7,8 and dissociates into monomers at a pH of 4,5 to 5,5.

2. The method of claim 1, wherein the peptide repeat is an octapeptide, pseudooctapeptide, hexapeptide or pseudohexapeptide.

3. The method of claim 2, wherein the octapeptide has a sequence selected from the group consisting of PHGGGWGQ (human), PHGGSWGQ (mouse) and PHGGGWSQ (rat), or is a pseudooctapeptide derived from said sequences, preferably selected from the group consisting of PHGGGGWSQ (various species), and PHGGGSNWGQ (marsupial).

4. The method of claim 2, wherein the hexapeptide has a sequence selected from the group consisting of PHNPGY (chicken), PHNPSY, PHNPGY (turtle) or is a pseudohexapeptide derived from said sequences.

5. The method according to any one of claims 1 to 4, wherein each of the peptide repeats comprises an N-terminal loop conformation connected to a C-terminal β-turn structure.

6. The method according to any one of claims 1 to 5, wherein the polypeptide comprises four identical octapeptides.

7. Use of a method according to any one of claims 1 to 6 for detecting human or animal prion proteins.

8. Use of a method according to any one of claims 1 to 6 for affinity purification and/or enrichment of said polypeptides.

9. Use of a method according to claims 7 or 8, wherein said polypeptide is immobilized on a solid phase.

10. Use of a method according to any one of claims 1 to 6 for chemical and/or physical sensor technology.

## Patentansprüche

1. Ein Verfahren zur reversiblen Aggregation und/oder Dissoziation von Polypeptiden, das den Schritt der Oligomerisierung eines Polypeptids bei einem pH-Wert von 6,2 bis 7,8 und/oder der Dissoziierung eines Polypeptidaggregats bei einem pH-Wert von 4,5 bis 5,5 in einer flüssigen Umgebung umfasst, wobei das Polypeptid **dadurch gekennzeichnet ist, dass**
(i) das Polypeptid eine oder mehrere sich wiederholende Peptidstrukturen umfasst, die sich aus Prionenproteinen ableiten,
(ii) und dass das Protein in Flüssigkeit bei einem pH-Wert von 6,2 bis 7,8 oligomerisiert und in Monomere bei einem pH-Wert von 4,5 bis 5,5 dissoziiert.

2. Das Verfahren von Anspruch 1, wobei das sich wiederholende Peptid ein Octapeptid, Pseudooctapeptid, Hexapeptid oder Pseudohexapeptid ist.

3. Das Verfahren von Anspruch 2, wobei das Octapeptid eine Sequenz aufweist, die aus der Gruppe ausgewählt ist, die aus PHGGGWGQ (Mensch), PHGGSWGQ (Maus) und PHGGGWSQ (Ratte) besteht, oder ein Pseudooctapeptid ist, das sich aus diesen Sequenzen ableitet, das vorzugsweise aus der Gruppe ausgewählt ist, die aus PHGGGGWSQ (verschiedene Spezies) und PHGGGSNWGQ (Beuteltier) besteht.

4. Das Verfahren von Anspruch 2, wobei das Hexapeptid eine Sequenz aufweist, die aus der Gruppe ausgewählt ist, die aus PHNPGY (Huhn), PHNPSY, PHNPGY (Schildkröte) besteht, oder ein Pseudohexapeptid ist, das sich aus diesen Sequenzen ableitet.

5. Das Verfahren gemäß einem der Ansprüche 1 bis 4, wobei jedes der sich wiederholenden Peptide eine N-terminale Schleifenkonformation umfasst, die an eine C-terminale β-turn-Struktur gebunden ist.

6. Das Verfahren gemäß einem der Ansprüche 1 bis 5, wobei das Polypeptid vier identische Octapeptide umfasst.

7. Verwendung eines Verfahrens gemäß einem der Ansprüche 1 bis 6 zum Nachweis von menschlichen oder tierischen Prionenproteinen.

8. Verwendung eines Verfahrens gemäß einem der Ansprüche 1 bis 6 zur Affinitätsaufreinigung und/oder Anreicherung der Polypeptide.

9. Verwendung eines Verfahrens gemäß den Ansprüchen 7 oder 8, wobei das Polypeptid auf einer festen Phase immobilisiert ist.

10. Verwendung eines Verfahrens gemäß einem der Ansprüche 1 bis 6 für eine chemische und/oder physikalische Sensortechnologie.

## Revendications

1. Procédé d'agrégation réversible et/ou de dissociation de polypeptides, comprenant l'étape consistant à oligomériser un polypeptide à un pH de 6,2 à 7,8 et/ou à dissocier un agrégat polypeptidique à un pH de 4,5 à 5,5 dans un environnement liquide, ledit polypeptide étant **caractérisé en ce que** :
(i) le polypeptide comprend une ou plusieurs structures peptidiques répétitives provenant de protéines prioniques ; et **en ce que**
(ii) la protéine s'oligomérise dans le liquide à un pH de 6,2 à 7,8 et se dissocie en monomères à un pH de 4,5 à 5,5.

2. Procédé selon la revendication 1, dans lequel la répétition peptidique est un octapeptide, un pseudo octapeptide, un hexapeptide ou un pseudo hexapeptide.

3. Procédé selon la revendication 2, dans lequel l'octapeptide a une séquence choisie dans le groupe constitué par PHGGGWGQ (homme), PHGGSWGQ (souris) et PHGGGWSQ (rat) ou est un pseudo octapeptide dérivé desdites séquences, de préférence choisi dans le groupe constitué par PHGGGGWSQ (espèces diverses) et PHGGGSNWGQ (marsupiaux).

4. Procédé selon la revendication 2, dans lequel l'hexapeptide a une séquence choisie dans le groupe constitué par PHNPGY (poulet), PHNPSY, PHNPGY (tortue) ou bien est un pseudo hexapeptide dérivé desdites séquences.

5. Procédé selon l'une quelconque des revendications 1 à 4, dans lequel chacune des répétitions peptidiques comprend une conformation en boucle N-terminale reliée à une structure en spire β C-terminale.

6. Procédé selon l'une quelconque des revendications 1 à 5, dans lequel le polypeptide comprend quatre octapeptides identiques.

7. Utilisation d'un procédé selon l'une quelconque des revendications 1 à 6 pour détecter des protéines prioniques humaines ou animales.

8. Utilisation d'un procédé selon l'une quelconque des revendications 1 à 6 pour la purification par affinité et/ou l'enrichissement desdits polypeptides.

9. Utilisation d'un procédé selon la revendication 7 ou la revendication 8, dans lequel ledit polypeptide est immobilisé sur une phase solide.

10. Utilisation d'un procédé selon l'une quelconque des revendications 1 à 6, pour une technologie de détection chimique et/ou physique.
